# EUROPEAN PATENT APPLICATION

(11) **EP 1 470 781 A2**
(43) Date of publication of application: **27.10.2004**
(21) Application number: 04252342.3
(22) Date of filing: 21.04.2004
(51) Int. Cl.: A61B 5/15, A61L 2/03, A61L 2/04, A61L 2/08, A61L 2/10, A61L 2/18

(54) **Lancet device and case therefor**

(30) Priority: 23.04.2003 JP 2003118738
(71) Applicant: MATSUSHITA ELECTRIC INDUSTRIAL CO., LTD., Kadoma-shi, Osaka 571-8501 (JP)
(72) Inventor: Nakayama, Hiroshi, Hirakata-shi Osaka 573-1114 (JP)
(74) Representative: Price, Paul Anthony King

(57) **Abstract**

A lancet device including: a puncture needle (5); a drive spring (2) adapted to move the puncture needle from a accommodated position to a lancing position; a trigger (4) adapted to release the drive spring from a compressed state to move the puncture needle from the accommodated position to the lancing position; a main casing (8) having space for accommodating the puncture needle and the drive spring and having a puncture hole (9) for guiding a tip of the puncture needle to an outside of the main casing; and a sterilizer (16) adapted to sterilize the puncture needle.

## Description

### BACKGROUND OF THE INVENTION

When sensing an object in a bodily fluid, a puncture needle is used to extract the bodily fluid from a living body. The puncture needle once used must be sterilized if it is reused. So far, the puncture needle has been sterilized by immersion in an antiseptic solution containing a chemical agent such as ethanol or by wiping with cotton impregnated with the antiseptic solution. Further, sterilization of the needle has been done by irradiation with an ultraviolet (UV) ray, heating or boiling. However, these sterilization methods are labor-consuming and cumbersome.

In order to sterilize the puncture needle with higher reliability, it is necessary to sterilize the puncture needle with γ rays or ethylene oxide gas. However, certain facilities are required to utilize such means, which is also labor-consuming and inefficient.

Therefore, it has been proposed to use a puncture needle, which is sterilized with γ rays or ethylene oxide gas and covered with a cap, and dispose the puncture needle after a single use (see the specification of USP 5,385,571).

### BRIEF SUMMARY OF THE INVENTION

An aspect of the present invention is to provide a lancet device provided with a mechanism which allows sterilization of a puncture needle in a labor-saving and effective manner, for example, before or after lancing operation. Another aspect of the present invention is to provide a case for a lancet device provided with a mechanism which allows labor-saving and effective sterilization of a puncture needle of a lancet device accommodated in the case.

The present invention relates to a lancet device comprising a puncture needle and a sterilizer for sterilizing the puncture needle. The present invention also relates to a case for a lancet device, the case comprising a sterilizer for sterilizing a puncture needle of a lancet device accommodated in the case.

Examples of the sterilizer may include, for example, a heater for heating the puncture needle, a radiation source for irradiating the puncture needle with an UV ray or an infrared ray or a chemical agent unit for applying an antiseptic solution to the puncture needle.

The heater may be a heating coil, an infrared laser, an LED (light-emitting diode) or the like. As the radiation source, a UV lamp, an infrared lamp, a laser or an LED emitting an UV or infrared ray may be used. The power consumption of the heater and the radiation source is preferably 1 mW or larger.

The chemical agent unit comprises an antiseptic solution or a porous body impregnated with the antiseptic solution. Examples of a chemical agent contained in the antiseptic solution include, for example, glutaral, sodium hypochlorite, povidone iodine, an aqueous solution containing iodine, ethanol, isopropanol, chlorhexidine, benzalkonium, phenol, cresol, gluconic acid, benzalkonium chloride, alkyldiaminoethylglycine hydrochloride, hydrogen peroxide aqueous solution, formaldehyde and the like. These may be used solely or in combination.

The puncture needle is sterilized by being inserted into the porous body impregnated with the antiseptic solution, for example. Examples of the porous body include a material which is flexible and capable of easily holding the solution, such as woven fabric, nonwoven fabric and a sponge.

The chemical agent unit preferably includes an indicator lamp for indicating a decrease in an amount of the antiseptic solution contained in the porous body and an antiseptic solution inlet for providing the porous body with the antiseptic solution.

If the lancet device has the sterilizer, the radiation source is preferably provided with a mechanism for automatically irradiating the puncture needle with an UV ray or an infrared ray for a certain period, e.g., 10 seconds or more, before or after lancing operation, for example. Further, the heater is preferably provided with a mechanism for automatically heating the puncture needle for a certain period, e.g., 10 seconds or more, before or after lancing operation, for example. The heater and the radiation source preferably come to rest when the lancet device enters the state ready for the lancing operation.

If the case for the lancet device has the sterilizer, the radiation source is preferably provided with a mechanism for automatically irradiating the puncture needle of the lancet device accommodated in the case with an UV ray or an infrared ray for a certain period, e.g., 10 seconds or more. Further, the heater is preferably provided with a mechanism for automatically heating the puncture needle of the lancet device accommodated in the case for a certain period, e.g., 10 seconds or more. The heater and the radiation source preferably come to rest while the lancet device is not accommodated in the case.

For example, the present invention relates to a lancet device comprising: a puncture needle; a drive spring for moving the puncture needle from an accommodated position to a lancing position; a trigger for releasing the drive spring from a compressed state to move the puncture needle from the accommodated position to the lancing position; a main casing having space for accommodating the puncture needle and the drive spring and having a puncture hole for guiding the tip of the puncture needle to the outside; and a sterilizer for sterilizing the puncture needle.

The sterilizer may be integrated with the main casing or fixed to a removable lid for covering the puncture hole in the main casing.

The sterilizer preferably comprises a circuit including a heating element and a switch for actuating the heating element, the switch being closed while the drive spring is being released.

The sterilizer preferably comprises a circuit including a radiation source for emitting an UV ray or an infrared ray and a switch for actuating the radiation source, the switch being closed while the drive spring is being released.

The sterilizer preferably comprises a porous body and an antiseptic solution impregnated in the porous body and the tip of the puncture needle is inserted into the porous body, for example, while the drive spring is being released.

The porous body is preferably configured so that it enters the puncture hole. In this case, the tip of the puncture needle may or may not project toward outside the puncture hole while the drive spring is being released.

The present invention further relates to a case for a lancet device having space for accommodating the lancet device and a sterilizer for sterilizing the puncture needle of the lancet device accommodated in the case.

The sterilizer preferably comprises a circuit including a heating element and a switch for actuating the heating element, the switch being closed while the lancet device is being accommodated in the case.

The sterilizer preferably comprises a circuit including a radiation source for emitting an UV ray or an infrared ray and a switch for actuating the radiation source, the switch being closed while the lancet device is being accommodated in the case.

The sterilizer preferably comprises a porous body and an antiseptic solution impregnated in the porous body and the tip of the puncture needle is inserted into the porous body while the lancet device is being accommodated in the case.

According to the lancet device of the present invention, the puncture needle is sterilized in a labor-saving and effective manner, for example, before or after the lancing operation. Further, according to the case for the lancet device of the present invention, the puncture needle is sterilized in a labor-saving and effective manner while the lancet device is being accommodated in the case.

While the novel features of the invention are set forth particularly in the appended claims, the invention, both as to organization and content, will be better understood and appreciated, along with other objects and features thereof, from the following detailed description taken in conjunction with the drawings.

### BRIEF DESCRIPTION OF THE SEVERAL VIEWS OF THE DRAWINGS

FIG. 1A is a view illustrating an inner structure of a lancet device according to Embodiment 1, with a drive spring being fixed.
FIG. 1B is a view illustrating the inner structure of the lancet device according to Embodiment 1, with the drive spring being released.
FIG. 2A is a view illustrating an inner structure of a lancet device according to Embodiment 2, with a drive spring being fixed and the end of the main casing being not yet engaged with the opening of the lid casing.
FIG. 2B is a view illustrating the inner structure of the lancet device according to Embodiment 2, with the drive spring being released and the end of the main casing being engaged with the opening of the lid casing.
FIG. 3A is a view illustrating an inner structure of a case according to Embodiment 2, with the lancet device being accommodated therein.
FIG. 3B is a view illustrating a major part of the case according to Embodiment 2, in which the lancet device is not yet accommodated.
FIG. 4A is a view illustrating an inner structure of a lancet device according to Embodiment 3, with a drive spring being fixed and the end of the main casing being not yet engaged with the opening of the lid casing.
FIG. 4B is a view illustrating the inner structure of the lancet device according to Embodiment 3, with the drive spring being released and the end of the main casing being engaged with the opening of the lid casing.
FIG. 5A is a view illustrating an inner structure of a case according to Embodiment 3, with the lanced device being accommodated therein.
FIG. 5B is a view illustrating a major part of the case according to Embodiment 3, in which the lancet device is not yet accommodated.
FIG. 6A is a view illustrating an inner structure of a lancet device according to Embodiment 4, with a drive spring being fixed and the end of the main casing being not yet engaged with the opening of the lid casing.
FIG. 6B is a view illustrating the inner structure of the lancet device according to Embodiment 4, with the drive spring being released and the end of the main casing being engaged with the opening of the lid casing.
FIG. 7 is a view illustrating an inner structure of a case according to Embodiment 4, with the lancet device being accommodated therein.
FIG. 8A is a view illustrating an inner structure of a lancet device according to Embodiment 5, with a drive spring being fixed.
FIG. 8B is a view illustrating the inner structure of the lancet device according to Embodiment 5, with the drive spring being released.

### DETAILED DESCRIPTION OF THE PREFERRED EMBODIMENTS

### Embodiment 1

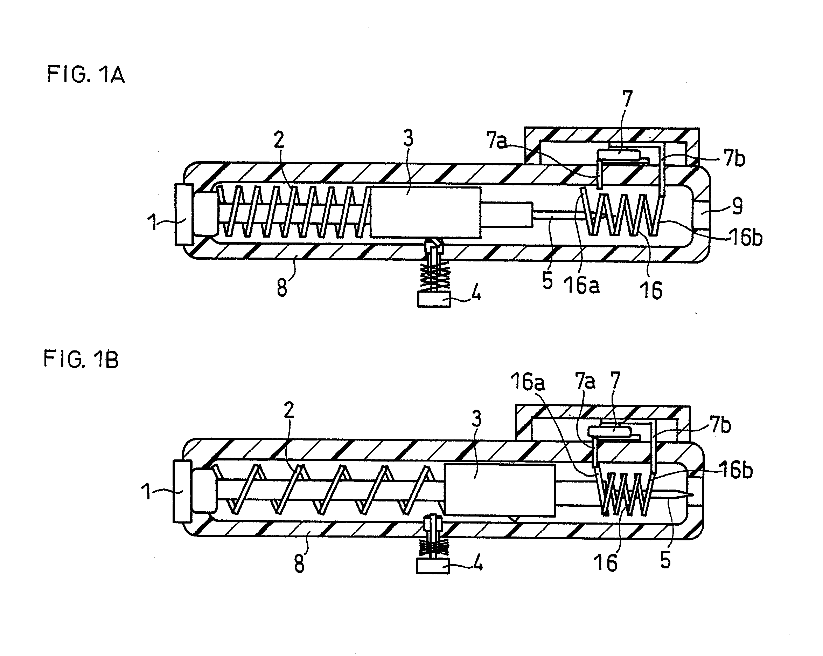

FIGs. 1A and 1B are sectional views illustrating an inner structure of a lancet device according to this embodiment. FIG. 1A shows a drive spring 2 fixed in a compressed state, while FIG. 1B shows the drive spring 2 in a released state.

The lancet device includes a puncture needle 5, a holder 3 for fixing the puncture needle 5, the drive spring 2 for moving the puncture needle 5 from an accommodated position to a lancing position and a trigger button 4 for releasing the drive spring 2 from the compressed state. The puncture needle 5 and the drive spring 2 are housed in a cylindrical main casing 8. At one end of the main casing 8, a puncture hole 9 is opened to guide the tip of the puncture needle 5 to the outside. At the other end thereof, a button 1 for compressing the drive spring 2 is provided. The drive spring 2 in the released state is compressed by pulling the button 1 before the lancing operation. By compressing the drive spring 2, the lancing device enters the state ready for the lancing operation. In this state, the trigger button 4 is pushed to release the drive spring 2. Thereby, the lancing operation is performed.

After the lancing operation, with the drive spring 2 being released, the puncture needle 5 is sterilized by a sterilizer. The sterilizer of the lancet device according to this embodiment has a heating element 16 comprising a resistance coil. An end 16a of the resistance coil contacts an external terminal 7a of a battery 7 while the drive spring 2 is being released, whereas the other end 16b of the resistance coil is always in contact with an external terminal 7b of the battery 7. That is, the heating element 16 and the battery 7 form a circuit. The end 16a of the resistance coil and the external terminal 7a of the battery 7 serve as a switch for actuating the heating element 16. The switch is closed with the drive spring 2 being released, and opened with the drive spring 2 being compressed.

According to the lancet device of this embodiment, the drive spring 2 is kept released for a while after the lancing operation, during which the puncture needle 5 is heat-sterilized by the heating element 16. Accordingly, there is no need of detaching the puncture needle 5 after the lancing operation to be soaked in a chemical solution or wiped with cotton impregnated with the chemical solution. That is, the puncture needle 5 is sterilized in a labor-saving and effective manner. The positional relationship between the puncture needle 5 and the heating element 16 is not limited to that shown in FIGs. 1A and 1B. The puncture needle 5 and the heating element 16 may be arranged closely to such a degree that the puncture needle 5 is raised in temperature.

### Embodiment 2

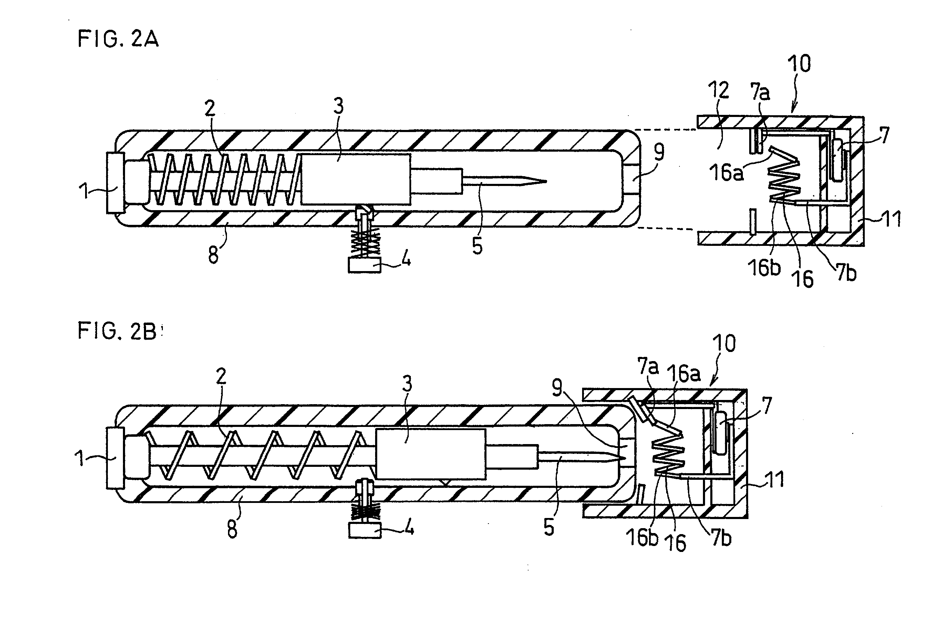

FIGs. 2A and 2B are sectional views illustrating an inner structure of a lancet device according to this embodiment. FIG. 2A shows a drive spring 2 fixed in a compressed state, while FIG. 2B shows the drive spring 2 in a released state.

The lancet device is structurally the same as that of Embodiment 1 except the difference in structure of the sterilizer. In this Embodiment, the sterilizer is installed in a removable lid 10 for covering the puncture hole 9 formed in the main casing 8.

The lid 10 includes a lid casing 11 having an opening 12 to be engaged with the end of the main casing 8 provided with the puncture hole 9. Inside the opening 12, a heating element 16 comprising a resistance coil and a battery 7 are installed. The heating element 16 and the battery 7 form a circuit, while an external terminal 7a of the battery 7 and an end 16a of the resistance coil form a switch for actuating the heating element 16.

In FIG. 2A, the end of the main casing 8 is not engaged with the opening 12 of the lid casing 11. In this state, the end 16a of the resistance coil is apart from the external terminal 7a of the battery 7. Referring to FIG. 2B, the end of the main casing 8 is engaged with the opening 12 of the lid casing 11. In this state, the end of the main casing 8 pushes the external terminal 7a of the battery 7 to bring it in contact with the end 16a of the resistance coil.

After the lancing operation, with the drive spring 2 being released, the lid 10 is attached to the end of the main casing 8. This state is maintained for a while, during which the puncture needle 5 is heat-sterilized by the heating element 16. The positional relationship between the puncture needle 5 and the heating element 16 is not limited to that shown in FIGs. 2A and 2B. The puncture needle 5 and the heating element 16 may be arranged closely to such a degree that the puncture needle 5 is raised in temperature. During the sterilization, the tip of the puncture needle 5 may be projected from the main casing 8 through the puncture hole 9.

Then, the following experiment was carried out.

The lancet device was formed as described above, with which lancing operation was performed to collect blood. Then, the lid 10 was attached to the end of the main casing 8 to heat-sterilize the puncture needle 5 at 1 W for 10 seconds. The tip of the sterilized puncture needle was brought into contact with a culture medium for determining viable cell count (manufactured by NISSHO CORPORATION). The culture medium was left stand at 37°C for 24 hours and then the viable cell count on the culture medium was determined. However, no bacteria were detected. Thus, it was ascertained that the heat-sterilization according to this embodiment is effective.

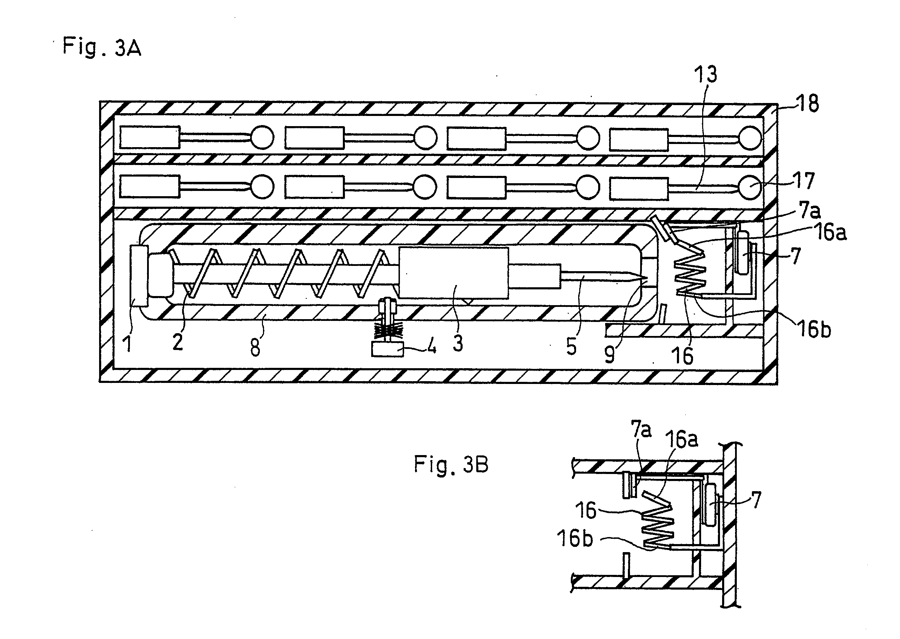

FIGs. 3A and 3B are sectional views illustrating an inner structure of a case for a lancet device having the same sterilization mechanism as described above.

The case has space for accommodating the lancet device and a sterilizer for sterilizing the puncture needle of the lancet device accommodated in the case. Referring to FIG. 3A, the case has the same heater as described in FIG. 2A and accommodates the lancet device therein. In this state, the external terminal 7a of the battery 7 is pushed by the end of the main casing 8 of the lancet device to contact with the end 16a of the resistance coil. In the state where the lancet device is not yet accommodated as shown in FIG. 3B, the end 16a of the resistance coil is apart from the external terminal 7a of the battery 7.

Due to the above-described structure, by accommodating the lancet device in the case, the switch for actuating the sterilizer is automatically closed to sterilize the puncture needle 5. The heating element 16 is preferably arranged close to the puncture hole 9 opened in the main casing 8 of the lancet device.

The case may be provided with space for carrying virgin spare puncture needles 13. The space may preferably have caps 17 for covering the tips of the spare puncture needles 13. In this case, the space is preferably arranged close to the heating element 16. While the sterilizer is working, the neighborhood of the sterilizer is also raised in temperature. Thereby, during the sterilization of the puncture needle 5 held by the lancet device, the spare puncture needles 13 before the lancing operation are also sterilized at the same time.

### Embodiment 3

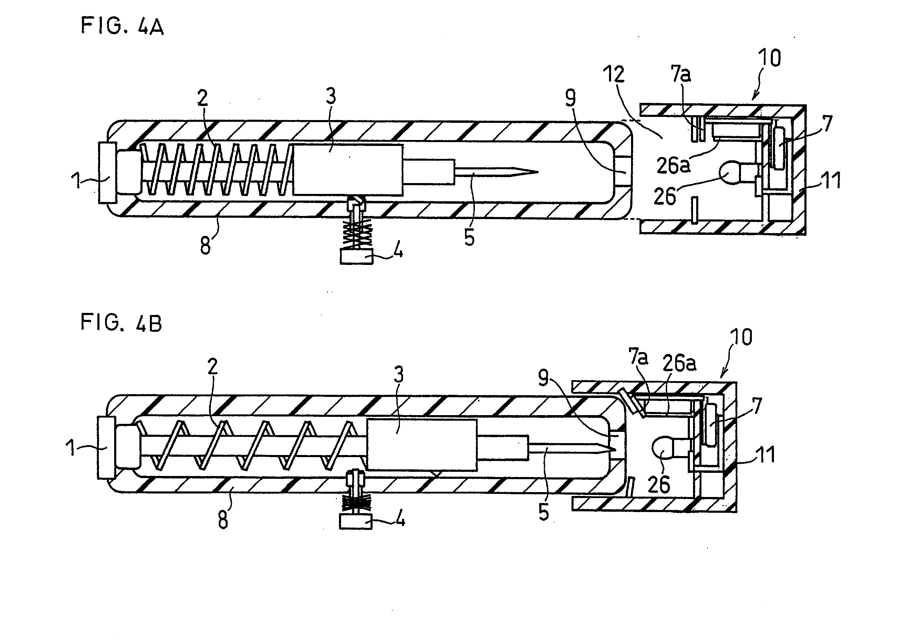

FIGs. 4A and 4B are sectional views illustrating an inner structure of a lancet device according to this embodiment. FIG. 4A shows a drive spring 2 fixed in a compressed state, while FIG. 4B shows the drive spring 2 in a released state.

The lancet device is structurally the same as that of Embodiment 2 except that a radiation source 26 comprising a UV lamp is used as the sterilizer. That is, when the end of the main casing 8 is not engaged with the opening 12 of the lid casing 11, an external terminal 26a of the radiation source 26 is apart from the external terminal 7a of the battery 7. On the other hand, if the end of the main casing 8 is engaged with the opening 12 of the lid casing 11, the external terminal 7a of the battery 7 is pushed by the end of the main casing 8 to contact with the external terminal 26a of the radiation source 26.

After the lancing operation, with the drive spring 2 being released, the lid 10 is attached to the end of the main casing 8. This state is maintained for a while, during which the puncture needle 5 is sterilized by an UV ray emitted from the radiation source 26. The positional relationship between the puncture needle 5 and the radiation source 26 is not limited to that shown in FIGs. 4A and 4B. The radiation source 26 may be arranged such that the tip of the puncture needle 5 is irradiated with the UV ray.

Then, the following experiment was carried out.

The lancet device was formed as described above, with which the lancing operation was performed to collect blood. Then, the lid 10 was attached to the end of the main casing 8 to sterilize the puncture needle 5 with an UV ray at 1 W for 30 seconds. The tip of the sterilized puncture needle 5 was brought into contact with a culture medium for determining viable cell count (manufactured by NISSHO CORPORATION). The culture medium was left stand at 37°C for 24 hours and then the viable cell count on the culture medium was determined. However, no bacteria were detected. Thus, it was ascertained that the sterilization by the UV ray according to this embodiment is effective.

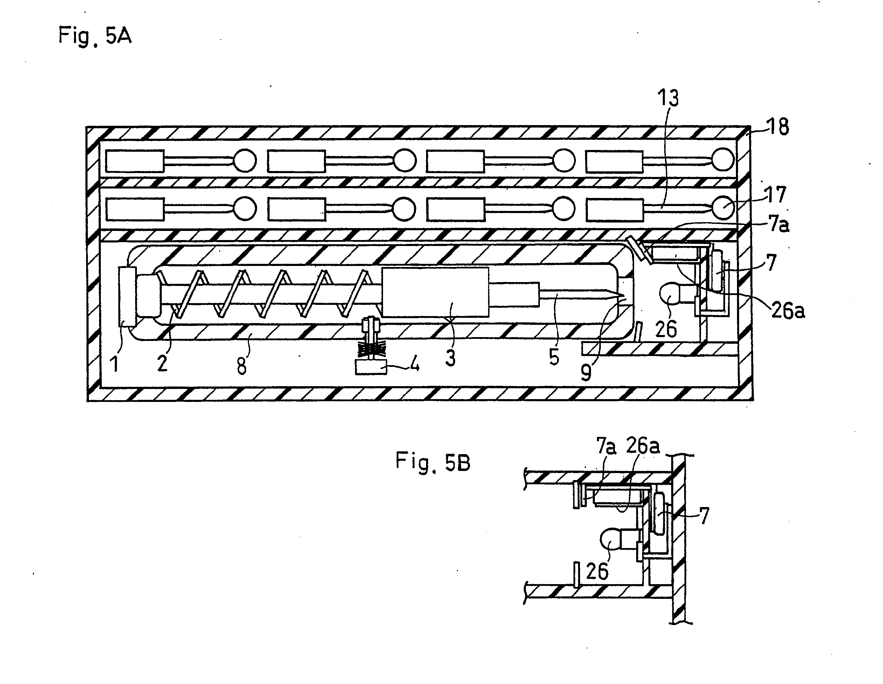

FIGs. 5A and 5B are sectional views illustrating an inner structure of a case for a lancet device having the same sterilizing mechanism as described above. The case is structurally the same as that of Embodiment 2 except the difference in structure of the sterilizer.

FIG. 5A shows a case having the same sterilizer as shown in FIGs. 4A and 4B comprising the UV lamp, with the lancet device being accommodated. In this state, the external terminal 7a of the battery 7 is pushed by the end of the main casing 8 of the lancet device to contact with the external terminal 26a of the radiation source 26. In the state where the lancet device is not yet accommodated as shown in FIG. 5B, the external terminal 26a of the radiation source 26 is apart from the external terminal 7a of the battery 7.

According to the above structure, the switch for actuating the sterilizer is automatically closed when the lancet device is accommodated in the case.

### Embodiment 4

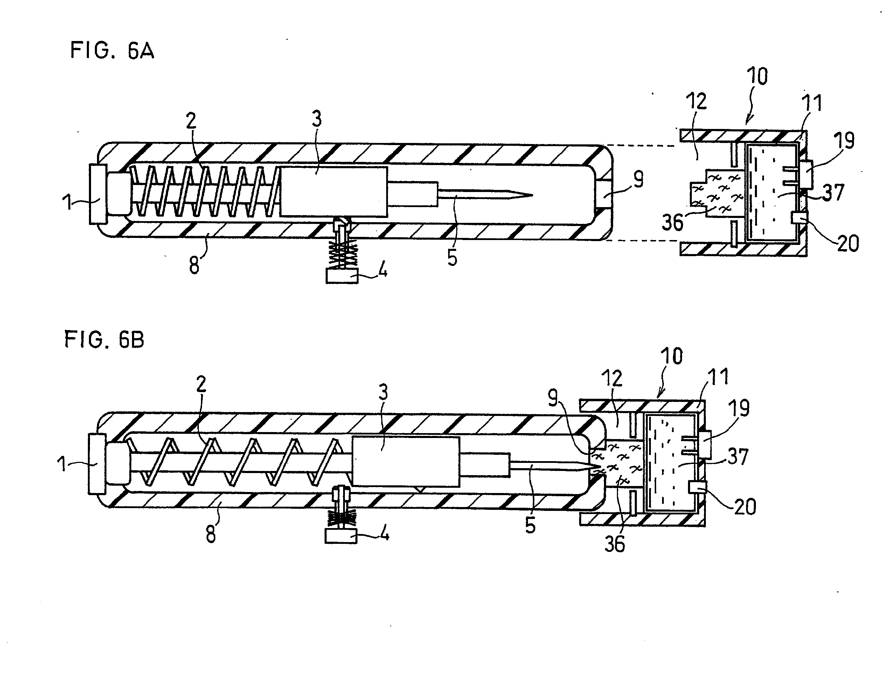

FIGs. 6A and 6B are sectional views illustrating an inner structure of a lancet device according to this embodiment. FIG.. 6A shows a drive spring 2 fixed in a compressed state, while FIG. 6B shows the drive spring 2 in a released state.

The lancet device is structurally the same as that of Embodiment 1 except the difference in structure of the sterilizer. In this embodiment, the sterilizer is installed in a removable lid 10 for covering the puncture hole 9 in the main casing 8.

The lid 10 comprises a lid casing 11, in which a porous body 36 and an antiseptic solution reservoir 37 are installed. The antiseptic solution reservoir 37 supplies the porous body 36 with the antiseptic solution. The porous body 36 is always impregnated with the antiseptic solution. The lid 10 has an opening 12 to be engaged with the end of the main casing 8 provided with the puncture hole 9. The porous body 36 is so arranged that it is opposed to the puncture needle 9 when the end of the main casing 8 is engaged with the opening 12 of the lid casing 11.

After the lancing operation, with the drive spring 2 being released, the lid 10 is attached to the end of the main casing 8. In this state, the tip of the puncture needle 5 is inserted into the porous body 36 and sterilized with the antiseptic solution.

The porous body 36 may be made of any material as long as the material is porous and capable of swelling in the antiseptic solution. For example, a sponge, woven fabric, nonwoven fabric and the like are preferably used. The porous body 36 and the antiseptic solution reservoir 37 may be connected, for example, by a porous material capable of transferring the antiseptic solution by capillary action.

As the antiseptic solution is consumed, the amount of the antiseptic solution contained in the antiseptic solution reservoir 37 decreases. Accordingly, it is preferable to provide the lid 10 with an indicator lamp 19 for indicating the decrease in amount of the antiseptic solution and an inlet 20 for introducing a supplemental antiseptic solution to the antiseptic solution reservoir 37.

Then, the following experiment was performed.

As the antiseptic solution, an aqueous solution containing 70 wt% of ethanol was prepared, which was introduced into the antiseptic solution reservoir 37 of the lid 10. The lancet device was formed as described above, with which the lancing operation was performed to collect blood. Then, the lid 10 was attached to the end of the main casing 8 to sterilize the puncture needle 5 with the antiseptic solution. Then, the tip of the puncture needle 5 is brought into contact with a culture medium for determining viable cell count (manufactured by NISSHO CORPORATION). The culture medium was left stand at 37°C for 24 hours and then the viable cell count on the culture medium was determined. However, no bacteria were detected. Thus, it was ascertained that the sterilization according to this embodiment is effective.

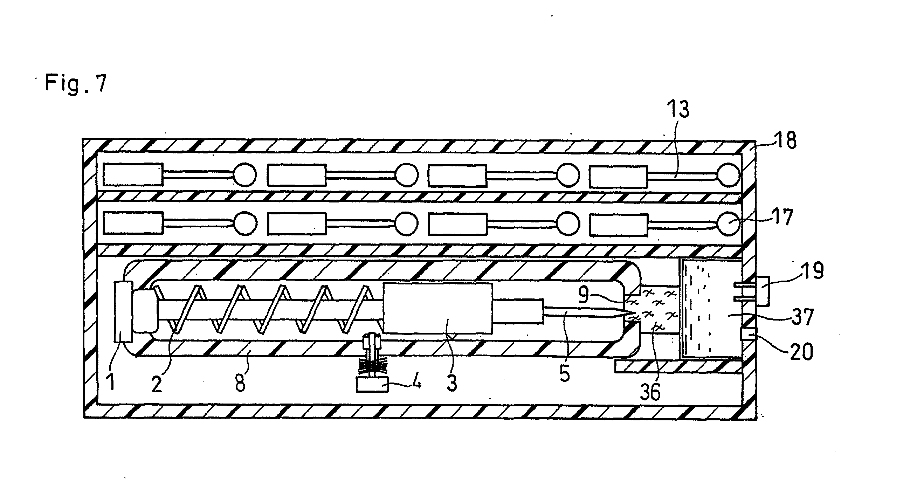

FIG. 7 is a view illustrating an inner structure of a case for a lancet device having the same sterilizing mechanism as described above, with a main body 18 shown in cross section. This case is structurally the same as that of Embodiment 2 except the difference in structure of the sterilizer.

Referring to FIG. 7, the lancet device is accommodated in the case having the same sterilizer as that shown in FIGs. 6A and 6B including the porous body 36 and the antiseptic solution reservoir 37. In this state, the tip of the puncture needle 5 is inserted into the porous body 36 and sterilized with the antiseptic solution. According to this structure, the puncture needle 5 is automatically sterilized when the lancet device is accommodated in the case.

### Embodiment 5

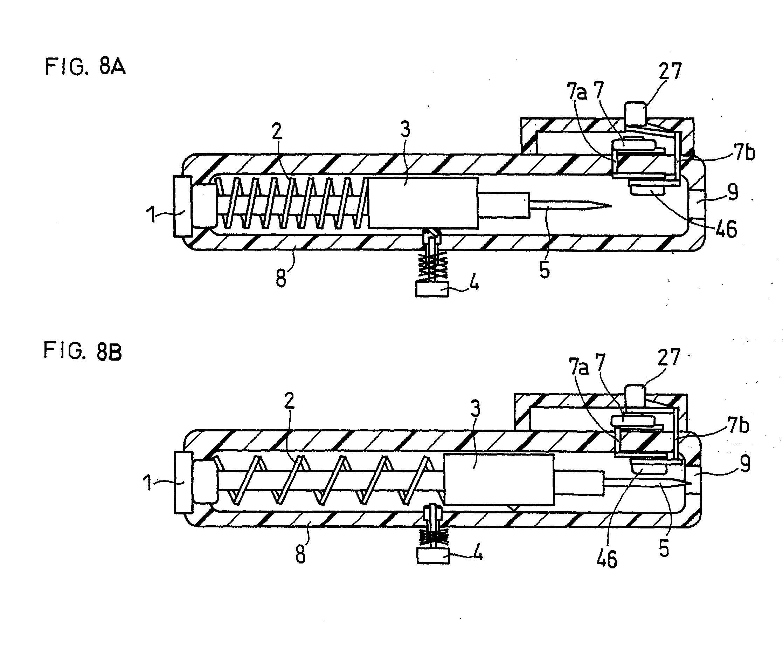

FIGs. 8A and 8B are sectional views illustrating an inner structure of a lancet device according to this embodiment. FIG. 8A shows a drive spring 2 fixed in a compressed state, while FIG. 8B shows the drive spring 2 in a released state. The lancet device is structurally the same as that of Embodiment 1 except the difference in structure of the sterilizer.

The sterilizer of the lancet device according to this embodiment has a radiation source 46 comprising a laser or an LED which emits an UV ray. In accordance with the ON/OFF state of a switch 27, electric current flows between the radiation source 46 and the battery 7. According to this structure, the puncture needle 5 can be sterilized at any time by merely turning the switch 27 on. If the switch 27 is turned on with the drive spring 2 being released, the puncture needle 5 comes closer to the radiation source 46. Accordingly, it is preferable to turn the switch 27 on with the drive spring 2 being released because the puncture needle 5 is sterilized more effectively.

As described above, the present invention allows sterilization of the puncture needle, for example, before or after lancing operation, in a labor-saving and effective manner. The lancet device and the case therefor according to the present invention are particularly convenient for reuse of a puncture needle once used or continuous lancing operation.

Although the present invention has been described in terms of the presently preferred embodiments, it is to be understood that such disclosure is not to be interpreted as limiting. Various alterations and modifications will no doubt become apparent to those skilled in the art to which the present invention pertains, after having read the above disclosure. Accordingly, it is intended that the appended claims be interpreted as covering all alterations and modifications as fall within the true spirit and scope of the invention.

## Claims

1. A lancet device used for performing a lancing operation, said lancet device comprising: a puncture needle; and a sterilizer arranged to sterilize said puncture needle.

2. The lancet device in accordance with claim 1, wherein said puncture needle is arranged to be sterilized by said sterilizer before or after the lancing operation.

3. The lancet device in accordance with claim 1 or 2, wherein said sterilizer is a heater arranged to heat said puncture needle, a radiation source arranged to irradiate said puncture needle with an ultraviolet ray or an infrared ray, or a chemical agent unit arranged to apply an antiseptic solution to said puncture needle.

4. The lancet device in accordance with claim 3, wherein said chemical agent unit comprises a porous body impregnated with the antiseptic solution and arranged such that said puncture needle is arranged to be insertable into said porous body.

5. The lancet device in accordance with claim 4, wherein said puncture needle is arranged to be insertable into said porous body before or after the lancing operation.

6. The lancet device in accordance with claim 4,
wherein said chemical agent unit comprises an indicator lamp arranged to indicate a decrease in an amount of the antiseptic solution contained in said porous body and an antiseptic solution inlet arranged to provide said porous body with the antiseptic solution.

7. The lancet device in accordance with any preceding claim, wherein said sterilizer is not actuated during the lancing operation.

8. A case for accommodating a lancet device, said case comprising: a sterilizer arranged to sterilize a puncture needle of the lancet device accommodated in said case.

9. The case for a lancet device in accordance with claim 8, wherein said sterilizer is a heater arranged to heat the puncture needle, a radiation source arranged to irradiate the puncture needle with an ultraviolet ray or an infrared ray, or a chemical agent unit arranged to apply an antiseptic solution to the puncture needle.

10. The case for a lancet device in accordance with claim 9, wherein said chemical agent unit comprises a porous body impregnated with the antiseptic solution and arranged such that the puncture needle is arranged to be insertable into said porous body while the lancet device is accommodated in said case.

11. The case for a lancet device in accordance with claim 10, wherein said chemical agent unit comprises an indicator lamp arranged to indicate a decrease in an amount of the antiseptic solution contained in said porous body and an antiseptic solution inlet arranged to provide said porous body with the antiseptic solution.

12. The case for a lancet device in accordance with any one of claims 8 to 11, wherein said sterilizer is not actuated before the lancet device is accommodated in said case.

13. A lancet device comprising: a puncture needle; a drive spring arranged to move said puncture needle from an accommodated position to a lancing position; a trigger arranged to release said drive spring from a compressed state to move said puncture needle from the accommodated position to the lancing position; a main casing having space for accommodating said puncture needle and said drive spring and having a puncture hole arranged to guide a tip of said puncture needle to an outside of said main casing; and a sterilizer arranged to sterilize said puncture needle.

14. The lancet device in accordance with claim 13, wherein said sterilizer is integrated with said main casing.

15. The lancet device in accordance with claim 13, wherein said sterilizer is fixed to a removable lid arranged to cover said puncture hole in said main casing.

16. The lancet device in accordance with claim 13, wherein said sterilizer comprises a circuit including a heating element and a switch arranged to actuate said heating element, said switch being closed while said drive spring is being released.

17. The lancet device in accordance with claim 13, wherein said sterilizer comprises a circuit including a radiation source arranged to emit an ultraviolet ray or an infrared ray and a switch arranged to actuate said radiation source, said switch being closed while said drive spring is being released.

18. The lancet device in accordance with claim 13, wherein said sterilizer comprises a porous body and an antiseptic solution impregnated in said porous body, wherein the tip of said puncture needle is arranged to be inserted into said porous body while said drive spring is being released.

19. The case for a lancet device in accordance with claim 8, wherein said sterilizer comprises a circuit including a heating element and a switch arranged to actuate said heating element, said switch being closed while the lancet device is being accommodated in said case.

20. The case for a lancet device in accordance with claim 8, wherein said sterilizer comprises a circuit including a radiation source arranged to emit an ultraviolet ray or an infrared ray and a switch arranged to actuate said radiation source, said switch being closed while the lancet device is being accommodated in said case.

21. A method for performing a lancing operation, said method comprising:
providing a puncture needle;
extracting fluid from a living body by using the puncture needle; and
sterilizing the puncture needle before or after performing said extracting fluid;
wherein said sterilizing is performed by heating the puncture needle, irradiating the puncture needle with an ultraviolet or infrared ray, or by applying an antiseptic solution to the puncture needle.

22. A method for accommodating a lancet device which includes a puncture needle, said method comprising:
housing the lancet device in a case; and
sterilizing the puncture needle of the lancet device accommodated in the case by heating the puncture needle, irradiating the puncture needle with an ultraviolet or infrared ray, or applying an antiseptic solution to the puncture needle.

23. A method for performing a lancing operation, said method comprising:
providing a puncture needle;
accommodating the puncture needle in a casing having a puncture hole arranged to guide a tip of the puncture needle to an outside of the casing;
moving the puncture needle from an accommodated position to a lancing position such that the tip of the puncture needle is moved to the outside of the casing;
extracting fluid from a living body by using the tip of the puncture needle; and
sterilizing the puncture needle before or after performing said extracting fluid.
